# EUROPÄISCHE PATENTSCHRIFT

(11) **EP 0 435 986 B1**
(45) Veröffentlichungstag und Bekanntmachung des Hinweises auf die Patenterteilung: **11.12.1996**
(21) Anmeldenummer: 90910546.2
(22) Anmeldetag: 20.07.1990
(51) Int. Cl.: A61B 10/00

(54) **VORRICHTUNG ZUR ENTNAHME VON GEWEBEPROBEN**
DEVICE FOR TAKING SAMPLES OF TISSUE
DISPOSITIF POUR LE PRELEVEMENT D'ECHANTILLONS TISSULAIRES

(30) Priorität: 22.07.1989 DE 3924291
(43) Veröffentlichungstag der Anmeldung: 10.07.1991
(73) Patentinhaber: HESKE, Norbert, D-82288 Kottgeisering (DE)
(72) Erfinder: HESKE, Norbert, D-82288 Kottgeisering (DE)
(74) Vertreter: Münich, Wilhelm, Dr.
(86) Internationale Anmeldenummer: DE9000549
(87) Internationale Veröffentlichungsnummer: WO9101112

(56) Entgegenhaltungen:
- EP-A- 0 173 653
- EP-A- 0 207 726
- EP-A- 0 238 461
- US-A- 4 735 215
- US-A- 4 881 551

## Beschreibung

### Technisches Gebiet

Die Erfindung bezieht sich auf eine Vorrichtung zur Entnahme von Gewebeproben gemäß dem Oberbegriff des Patentanspruchs 1. Derartige Vorrichtungen werden auch als Biopsie-Vorrichtungen bezeichnet.

### Stand der Technik

Bei bekannten Vorrichtungen gemäß dem Oberbegriff des Patentanspruchs 1 wird zunächst eine Biopsie-Nadel, die in ihrem distalen Endbereich eine Ausnehmung aufweist, in das Gewebe eingeführt, aus dem eine Gewebeprobe entnommen werden soll. Anschließend wird über die Biopsie-Nadel ein Außenrohr geschoben, das eine kleine, in der Ausnehmung befindliche Gewebeprobe von dem Gewebe abtrennt. Anschließend wird die Biopsie-Nadel zusammen mit dem über sie geschobenen Außenrohr aus dem Gewebe entfernt.

Nachteilig bei diesen bekannten Biopsie-Vorrichtungen gemäß dem Oberbegriff des Patentanspruchs 1 ist, daß der Durchmesser der Biopsie-Nadel einen bestimmten Wert nicht unterschreiten kann, da ansonsten die zum Einführen der Nadel in das Gewebe erforderliche Stabilität nicht mehr gegeben wäre. Damit weist notwendigerweise auch das die Biopsie-Nadel umgebende Außenrohr einen vergleichsweise großen Durchmesser auf.

Aus der europäischen Patentanmeldung 0 238 461 geht eine gattungsgemäße Vorrichtung zur Gewebsprobenentnahme hervor. Die kinematisch auf Schlittelelementen vorgesehenen, miteinander gekoppelten Biopsienadeln werden jeweils durch eine, die Schlittenelemente antreibende, Feder bewegt. Die Kinematik der Biopsienadel erfolgt dabei derart, daß beide Nadeln zunächst manuell in das zu untersuchende Gewebe eingebracht werden müssen. Durch Betätigung der gespannten Feder erfolgt sodann ein Vorschnellen der inneren Nadel, die für die Gewebeaufnahme eine entsprechende Ausnehmung aufweist. Danach wird die äußere Hohlkanüle, an deren distalen Ende eine Schneidfläche vorgesehen ist, über die innere Nadel geführt, so daß Gewebematerial, das sich in der Ausnehmung abgesenkt hat, abgeschnitten wird.

Ein vom Bewegungsablauf dem Erfindungsgegenstand näher kommender, jedoch vollständig manuell zu betätigender, Biopsienadelmechanismus geht aus der europäischen Patentanmeldung 0 207 726 hervor. Auch bei dieser Biopsievorrichtung sind zwei ineinander geführte Nadeln vorgesehen, die wie vorstehend beschrieben eine Gewebeentnahme ermöglichen.

Den bekannten Biopsievorrichtungen haftet jedoch der Nachteil an, daß zumindest der Einbringvorgang bei der Biopsievorrichtung gemäß der erstgenannten Druckschrift manuell vorgenommen werden muß. Das Biopsiegerät gemäß der zweitgenannten Druckschrift ist sogar für einen ausschließlichen manuellen Betrieb vorgesehen.

Die manuelle Einführung derartiger Biopsienadeln in den zu untersuchenden Körperbereich kann jedoch aufgrund der nur langsamen Schnittgeschwindigkeit, die durch das manuelle Einführen bedingt ist, irreversible Gewebeirritationen hervorrufen. Darüberhinaus können im Gewebe vorhandene härtere Bereiche die Biopsienadel zum Auswandern aus der originären Vorschubsrichtung ableiten und dadurch ebenso zu ungewollten Gewebeirritationen führen.

Hinzu kommt, daß Biopsienadeln im Durchmesser so klein wie möglich ausgebildet werden sollen, um Gewebeverletzungen, die unabdingbar mit einem Biopsieeingriff verbunden sind, zu minimieren. Dies hat jedoch die Folge, daß dünne Nadelsysteme nur eine geringe Längsstabilität aufweisen.

Der Erfindung liegt die Aufgabe zugrunde, eine Biopsie-Vorrichtung, d. h. eine Vorrichtung zur Entnahme von Gewebeproben anzugeben, die die Verwendung sehr dünner Biopsienadeln erlaubt und darüberhinaus das zu untersuchende Gewebematerial möglichst schont.

Eine erfindungsgemäße Lösung dieser Aufgabe ist im Patentanspruch 1 gekennzeichnet. Weiterbildungen der Erfindung sind Gegenstand von Unteransprüchen.

Erfindungsgemäß wird eine Vorrichtung zur Entnahme von Gewebeproben mit einer Biopsie-Kanüle, die ein Nadelelement mit einer Ausnehmung im distalen Endbereich und ein das Nadelelement umgebendes Außenrohr aufweist, und die eine Einbringeinheit in das Gewebe, aus dem Proben entnommen werden sollen, einbringt, mit zwei koaxial verschiebbaren Schlittenelementen, von denen das in Einbringrichtung hintere Schlittenelement das Nadelelement trägt, und das in Einbringrichtung vordere Schlittenelement ein weiteres, gegen das vordere Schlittenelement verschiebbares Schlittenelement trägt, das das Außenrohr trägt, derart weitergebildet, daß die Einbringeinheit durch Federkraftbeaufschlagung der Schlittenelemente, selbsttätig das Nadelelement und das Außenrohr gemeinsam in das Gewebe in einer Stellung einbringt, in der das Außenrohr über die Ausnehmung geschoben ist, nach Beendigung des Einbringvorgangs das Außenrohr hinter die Ausnehmung des Nadelelements zurückzieht, und anschließend das Außenrohr wieder über die Ausnehmung im Nadelelement schiebt.

Der Erfindung liegt die Idee zugrunde alle für die Gewebeentnahme notwendigen Nadelbewegungen Federkraft zu beaufschlagt, so daß durch eine einmalige Auslösung der Biopsievorrichtung die einzelnen Biopsienadelbewegungen sehr schnell, selbsttätig hintereinander ablaufen.

Eine derartige Federkraftbeaufschlagung führt zu einer enormen Beschleunigung der einzelnen Nadeln, die mit hoher Geschwindigkeit in das Gewebe eindringen und aufgrund ihrer hohen Geschwindigkeit eine entsprechend große Längsstabilität erfahren. Durch die hohe Wucht der Nadelbewegungen werden möglicherweise vorhandene Widerstände im Gewebe problemlos durchdrungen. Nadeldeviationen aus der ursprünglichen Bewegungsrichtung können auf diese Weise vermieden werden.

Überdies bringt die Einbringeinheit das Nadelelement nicht alleine, sondern gemeinsam mit dem Außenrohr in das Gewebe in einer Stellung ein, in der das Außenrohr über die Ausnehmung geschoben ist. Erst nach Beendigung des Einbringvorgangs wird das Außenrohr hinter die Ausnehmung im Nadelelement zurückgezogen. Hierdurch schiebt sich das Gewebe, aus dem eine Gewebeprobe entnommen werden soll, aufgrund der "elastischen Rückstell-kraft", die durch die Verdrängung des Gewebes durch die Kanüle hervorgerufen wird, in die Ausnehmung vor. Anschließend wird das Außenrohr wieder über die Ausnehmung im Nadelelement, d.h. der Biopsie-Nadel geschoben. In dieser Stellung wird dann die Biopsie-Kanüle entnommen.

Da bei der erfindungsgemäßen Vorrichtung das Nadelelement gemeinsam mit dem Außenrohr in das Gewebe eingebracht wird, ist für die Stabilität der Biopsie-Kanüle nicht allein das Nadelelement, sondern das Nadelelement zusammen mit dem umgebenden Außenrohr ausschlaggebend. Das eigentliche Nadelelement kann damit wesentlich dünner als bei herkömmlichen Biopsie-Vorrichtungen ausgeführt werden. Im Prinzip ist es sogar ausreichend, daß die gesamte Biopsie-Kanüle den gleichen Außendurchmesser wie die Biopsie-Nadel bei herkömmlichen Vorrichtungen hat, um eine mit herkömmlichen Vorrichtungen vergleichbare Stabilität der Kanüle beim Einbringvorgang zu erreichen.

Erfindungsgemäß weist die Einbringeinheit zwei koaxial verschiebbare Schlittenelemente auf, von denen das in Einbringrichtung hintere Schlittenelement das Nadelelement trägt, und das in Einbringrichtung vordere Schlittenelement ein weiteres, gegen das vordere Schlittenelement verschiebbares Schlittenelement trägt, das das Außenrohr trägt. Durch diese erfindungsgemäße Ausbildung mit insgesamt drei gemeinsam bzw. relativ zueinander verschiebbaren Schlittenelementen kann die Einbringeinrichtung den erfindungsgemäß vorgesehenen Bewegungsablauf mit gegebenenfalls einstellbaren Weglängen, in jedem Fall jedoch reproduzierbar ausführen.

In den Ansprüchen 2 bis 4 sind besonders einfache Ausgestaltungen des erfindungsgemäßen Federantriebs angegeben, die nicht nur ein einfaches "Spannen" der Einbringeinheit, sondern auch ein einfaches Einstellen der jeweiligen Weglängen durch Anschläge erlauben (Anspruch 5).

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1a bis 1d: einen schematischen durch eine erfindungsgemäße Einbringeinheit in verschiedenen Arbeitsstellungen,
- Fig. 2: die Befestigung einer Biopsie-Kanüle an einer Einbringeinheit gemäß Figur 1,
- Fig. 3a bis 3d: die jeweilige Stellung der Biopsie-Kanüle bei den in den Figuren 1a bis 1d gezeigten Stellungen der Einbringeinheit, und

### Darstellung eines Ausführungsbeispiels

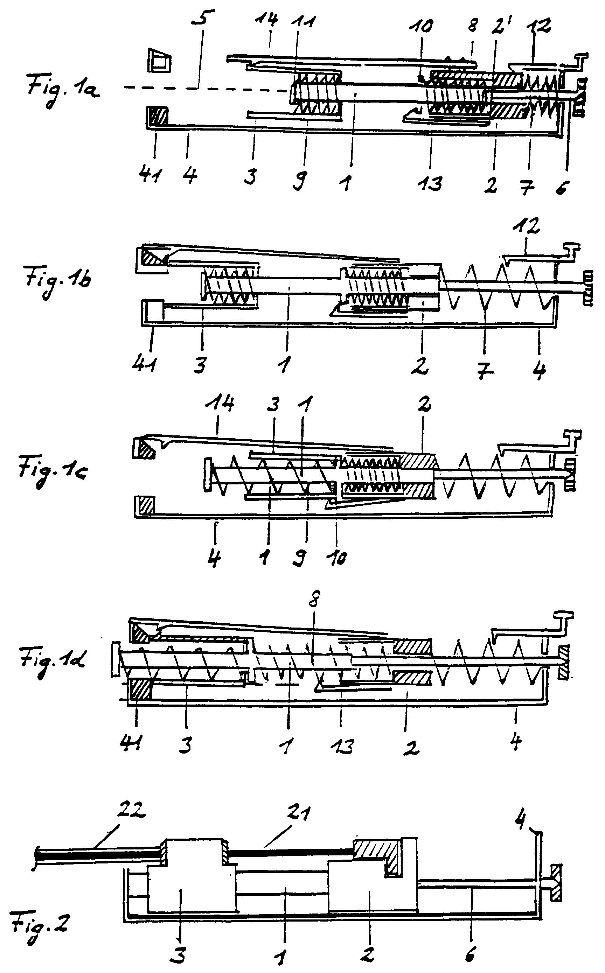

Die in den Figuren 1 und 2 dargestellte Einbringeinheit für Biopsie-Kanülen weist drei Schlittenelemente (i.f. einfach Schlitten) 1 bis 3 auf, die in einem Gehäuse 4 längs einer gemeinsamen Achse 5 verschiebbar gelagert sind. Zur Vereinfachung der Darstellung ist von den Lagerelementen lediglich ein Stiftelement 6 dargstellt, auf dem der Schlitten 1 in Richtung der Achse 5 verschiebbar gelagert ist.

Zum Antrieb der verschiedenen Schlitten sind Druckfedern 7 bis 9 vorgesehen, von denen die Feder 7 zwischen dem Gehäuse 4 und der hinteren Stirnseite des Schlittens 2, die Feder 8 zwischen einem Anschlag 10 am Schlitten 1 und einer Anlagefläche 2' am Schlitten 2 und die Feder 9 zwischen einem weiteren Anschlag 11 am Schlitten 1 und dem Schlitten 3 eingespannt ist.

Ferner sind als Auslöser dienende Sperrklinken 12 bis 14 vorgesehen, von denen die Sperrklinke 12 am Gehäuse und am die Sperrklinken 13 und 14 am Schlitten 2 gelagert sind. Die Sperrklinken 12 und 14 greifen in entsprechende Ausnehmungen an den Schlitten 2 bzw. 3 ein, während die Sperrklinke 13 am Anschlag 10 des Schlitten 1 anliegt.

Figur 2 zeigt, daß am Schlitten 2 eine Biopsie-Nadel 21 und am Schlitten 3 ein Außenrohr 22 einer Biopsie-Kanüle angebracht ist. Die Verbindung zwischen den Elementen 21 und 22 der Biopsie-Kanüle mit den Schlittenelementen 2 und 3 ist in Figur 2 exemplarisch als formschlüssige Verbindung dargestellt. Selbstverständlich sind auch andere Verbindungen möglich, solange sie nur ein leichtes Anbringen der Biopsie-Kanüle an der Einbringeinheit gestatten.

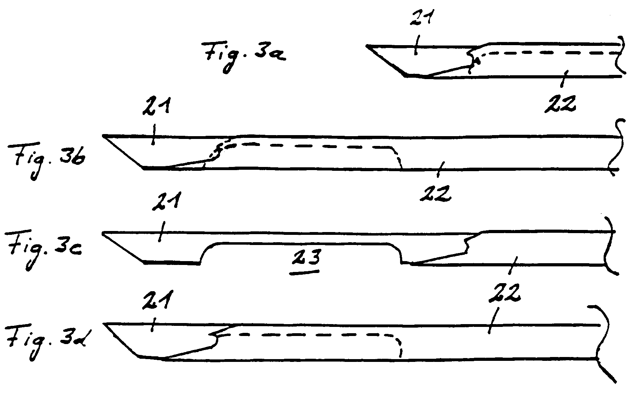

Im folgenden soll die Funktionsweise der erfindungsgemäßen Einbringeinheit unter Bezugnahme auf die Figuren 1a bis 1d sowie die Figuren 3a bis 3d, die die entsprechende Stellung der Biopsie-Kanüle zeigen, näher erläutert werden.

In dem in Figur 1a gezeigten Grundzustand vor dem Auslösen des Einbringvorgangs sind die Druckfedern 7 bis 9 gespannt und die Schlittenelemente 1 bis 3 durch die entsprechenden Sperrhaken bzw. Auslöser 12 bis 14 festgelegt. Entsprechend ist die Kanüle zurückgezogen, wobei das Außenrohr 22 die Ausnehmung 23 in der Biospienadel 21 überdeckt (Fig.3a).

Der Entnahmevorgang wird dadurch gestartet, daß der Sperrhaken bzw. Auslöser 12 betätigt wird. Hierdurch werden die Schlitten 1 bis 3 durch die Feder 7 in Richtung der Achse 5 gemeinsam verschoben. Der Verschiebeweg der drei Schlitten ist durch einen Anschlag 41 am Gehäuse 4 begrenzt; dieser Anschlag kann gegebenenfalls ein einstellbarer Anschlag sein, so daß die Vorschubbewegung beim Einbringen der Biopsie-Kanüle 21 und 22 einstellbar ist.

Figur 1b zeigt den Zustand der Einbringeinheit nach dem Einbringen der Biopsie-Kanüle. Figur 3b zeigt das distale Ende der Biopsie-Kanüle in diesem Zustand. Wie Figur 3b zu entnehmen ist, ist aufgrund der gemeinsamen Bewegung der einzelnen Elemente der Kanüle das Außenrohr 22 weiterhin soweit über die Nadel 21 geschoben, daß die Ausnehmung 23 in der Nadel 22 bedeckt ist.

In diesem Zustand kann nun - automatisch gesteuert durch entsprechende Steuerungselemente, beispielsweise eine nicht dargestellte Kurvenbahn am Gehäuse 4, oder von Hand - der Auslöser 14 betätigt werden. Hierdurch wird der Schlitten 3 durch die Kraft der Feder 9 in Richtung auf den Anschlag 10 bewegt.

Figur 1c zeigt das Ergebnis dieser Bewegung für die Einbringeinheit. Figur 3c zeigt, daß durch die Bewegung des Schlittenelements 3 relativ zu den Schlitten 1 und 2 das Außenrohr 22 hinter die Ausnehmung 23 in der Biopsie-Nadel rückgezogen wird.

Anschließend wird - ebenfalls selbsttätig beispielsweise durch eine entsprechende Kurvenbahn oder von Hand - der Auslöser 13 betätigt. Die Feder 8 verfährt damit den Schlitten 1 zusammen mit dem auf ihm festgelegten Schlitten 3 gegen den Anschlag 41, während der Schlitten 2 ortsfest bleibt (Figur 1d). Durch diese Bewegung wird das Außenrohr 22 wieder über die Ausnehmung 23 in der Nadel 21 geschoben (Figur 3d). Die Vorschubbewegung des Außenrohres 22 trennt das durch die Elastizität des Gewebes in die Ausnehmung 23 vorgeschobene Gewebe von dem restlichen Gewebe ab, so daß in der Ausnehmung 23 eine kleine Gewebeprobe verbleibt, die beispielsweise durch eine entsprechende Bewegung der Einbringeinheit nach rückwärts, der die aus der Nadel 21 und dem Außenrohr 22 bestehende Biopsie-Kanüle als Einheit folgt, entnommen werden kann.

Vorstehend ist die Erfindung anhand eines Ausführungsbeispieles ohne Beschränkung des allgemeinen Erfindungsgedankens beschrieben worden.

## Patentansprüche

1. Vorrichtung, zur Entnahme von Gewebeproben, mit einer Biopsie-Kanüle, die ein Nadelelement (21) mit einer Ausnehmung (23) im distalen Endbereich und ein das Nadelelement umgebendes Außenrohr (22) aufweist und die eine Einbringeinheit in das Gewebe, aus dem Proben entnommen werden sollen, einbringt, und mit zwei koaxial verschiebbaren Schlittenelementen (1,2), von denen das in Einbringrichtung hintere Schlittenelement (2) das Nadelelement (21) trägt und das in Einbringrichtung vordere Schlittenelement (1) ein weiteres, gegen das vordere Schlittenelement verschiebbares Schlittenelement (3) trägt, das das Außenrohr (22) trägt,
dadurch **gekennzeichnet**, daß die Einbringeinheit so ausgestaltet ist, daß sie durch Federkraftbeaufschlagung der Schlittenelemente (1,2), selbsttätig das Nadelelement (21) und das Außenrohr (22) gemeinsam in das Gewebe in einer Stellung einbringt, in der das Außenrohr über die Ausnehmung (23) geschoben ist, nach Beendigung des Einbringvorgangs das Außenrohr hinter die Ausnehmung des Nadelelements zurückzieht, und anschließend das Außenrohr wieder über die Ausnehmung im Nadelelement schiebt.

2. Vorrichtung nach Anspruch 1,
dadurch **gekennzeichnet**, daß eine erste Feder (7) die beiden koaxial verschiebbaren Schlittenelemente (1,2) in Einbringrichtung beaufschlagt, und daß die beiden Schlittenelemente in der Stellung, in der die Feder gespannt ist, durch ein lösbares Sperrelement (12) festlegbar ist.

3. Vorrichtung nach Anspruch 2,
dadurch **gekennzeichnet**, daß das in Einbringrichtung vordere Schlittenelement (1) einen Anschlag (1) für das weitere Schlittenelement (3) aufweist, gegen den das weitere Schlittenelement durch die Kraft einer zweiten Feder (9) nach Lösen eines Sperrelements (14) bewegbar ist.

4. Vorrichtung nach Anspruch 3,
dadurch **gekennzeichnet**, daß eine dritte Feder (8) vorgesehen ist, die zwischen beiden koaxial verschiebbaren Schlittenelemente (1,2) eingespannt ist, und die das vordere Schlittenelement (1) zusammen mit dem weiteren Schlittenelement (3) nach dem Lösen eines Sperrelements (13) derart in Einbringrichtung der Kanüle vorschiebt, daß das weitere Schlittenelement an die Stelle vorgeschoben wird, an der es sich vor dem Entspannen der zweiten Feder (9) befunden hat.

5. Vorrichtung nach einem der Ansprüche 1 bis 4,
dadurch **gekennzeichnet**, daß die Verschiebung der Schlittenelemente gegen einstellbare Anschläge (10,41) erfolgt.

## Claims

1. Device for tissue sampling, comprising a biopsy cannula including a needle element (21) with a cavity (23) in the distal terminal zone and an exterior tube (22) surrounding said needle element, and inserting an inserting unit into the tissue which samples are to be taken from, and comprising two coaxially displaceable slide elements (1, 2) whereof the slide element (2) which is rearward in the inserting direction carries said needle element (21) and the slide element (1) which is forward in the inserting direction carries a further slide element (3) which is displaceable relative to said forward slide element and which carries said exterior tube (22),
**characterized in** that said inserting unit is so designed that it introduces said needle element (21) and said exterior tube (22) jointly into the tissue automatically, by spring action produced on said slide elements (1, 2), into a position in which said exterior tube is pushed over said cavity (23), and that it retracts said exterior tube behind said cavity of said needle element upon termination of the inserting operation, and subsequently pushes said exterior tube again over said cavity in said needle element.

2. Device according to Claim 1,
**characterized** in that a first spring (7) biases said two coaxially displaceable slide elements (1, 2) in the inserting direction, and that said two slide elements are adapted to be arrested by a releasable locking element (12) in the position in which said spring is biased.

3. Device according to Claim 2,
**characterized** in that said slide element (1) which is forward in the inserting direction includes a stop (1) for said further slide element (3) toward to which said further slide element is movable under the action of a second spring (9) following the release of a locking element (14).

4. Device according to Claim 3,
**characterized** in that a third spring (8) is provided which is biased between both coaxially displaceable slide elements (1, 2) and which, following the release of a locking element (13), advances said forward slide element (1) together with said further slide element (3) in the cannula-inserting direction such that said further slide element is advanced to the position where it was located prior to the expansion of said second spring (9).

5. Device according to any of Claims 1 to 4,
**characterized** in that the displacement of said slide elements is performed toward adjustable stops (10, 41).

## Revendications

1. Dispositif pour le prélèvement d'échantillons de tissus, comprenant une canule bioptique à un élément d'aiguille (21) prévu d'un creux (23) dans la zone d'extrémité distale, et à un tube extérieur (22) qui entoure ledit élément d' aiguille, et qui comprend une unité d'introduction d'une unité d'introduction dans le tissu dont on veut prélever des échantillons, et qui comprend deux éléments coulisseaux (1, 2) à déplacement coaxial, dont le élément coulisseau (2), qui se trouve en arrière en sens d'introduction, porte ledit élément d'aiguille (21), et dont le élément coulisseau qui se trouve en avant en sens d'introduction, porte un élément coulisseau ultérieur (3), qui est déplaçable relativement audit élément coulisseau avant et qui porte ledit tube extérieur (22),
**caractérisé en** ce que ladite unité d'introduction est conçue de façon qu'elle introduit ledit élément d' aiguille (21) et ledit tube extérieur (22) en commun dans le tissu de façon automatisée, grâce à l'action de ressort produite sur lesdits éléments coulisseaux (1, 2), dans une position où ledit tube extérieur est poussé sur ledit creux (23), et en ce qu'elle retire ledit tube extérieur en arrière dudit creux dudit élément d'aiguille après I'achèvement de l'opération d'introduction, et ensuite pousse ledit tube extérieur de nouveau sur ledit creux dans ledit élément d'aiguille.

2. Dispositif selon la revendication 1,
**caractérisé** en ce qu'un premier ressort (7) rappelle lesdits deux éléments coulisseaux (1, 2) à déplacement coaxial en sens d'introduction, et en ce que lesdits deux éléments coulisseaux sont prévus à être bloqués moyennant un élément d' arrêt mobile (12) dans la position où ledit ressort est tendu.

3. Dispositif selon la revendication 2,
**caractérisé** en ce que ledit élément coulisseau (1), qui se trouve en avant en sens d'introduction, comprend une butée (1) pour ledit élément coulisseau ultérieur (3), contre lequel ledit élément coulisseau ultérieur se peut mouvoir sous l'action d'un deuxième ressort (9) après le desserrage d'un élément d'arrêt (14).

4. Dispositif selon la revendication 3,
**caractérisé** en ce qu'un troisième ressort (8) est prévu, qui est serré entre les deux éléments coulisseaux (1, 2) à déplacement coaxial, et qui, après le desserrage d'un élément d'arrêt (13), pousse ledit élément coulisseau (1) avant, ensemble avec ledit élément coulisseau ultérieur (3), en avant en sens d'introduction de la canule, de façon que ledit élément coulisseau ultérieur soit avancé au point où il se trouvait avant le relâchement dudit deuxième ressort (9).

5. Dispositif selon une quelconque des revendications 1 à 4,
**caractérisé** en ce que le déplacement desdits éléments coulisseaux se fait contre des butées ajustables (10, 41).
